# EUROPEAN PATENT SPECIFICATION

(11) **EP 1 094 804 B1**
(45) Date of publication and mention of the grant of the patent: **05.10.2005**
(21) Application number: 99935443.4
(22) Date of filing: 08.07.1999
(51) Int. Cl.: A61K 31/197, A61P 29/00, A61P 25/04

(54) **THE TREATMENT OF RENAL COLIC WITH GABA ANALOGS**
BEHANDLUNG VON NIERENKOLIK MIT GABA-ANALOGEN
TRAITEMENT DE COLIQUES NEPHRETIQUES AU MOYEN D'ANALOGUES DU GABA

(30) Priority: 09.07.1998 US 92167 P
(43) Date of publication of application: 02.05.2001
(73) Proprietor: Warner-Lambert Company LLC, NJ 07950 (US)
(72) Inventor: ANGELLO, James, T., Belle Mead,NJ 08502 (US)
(74) Representative: Mansmann, Ivo
(86) International application number: PCT/US1999/015387
(87) International publication number: WO 2000/002547

(56) References cited:
- WO-A-98/03167
- FIELD M J ET AL: "GABAPENTIN (NEURONTIN) AND S-(+)-3-ISOBUTYLGABA REPRESENT A NOVEL CLASS OF SELECTIVE ANTIHYPERALGESIC AGENTS" BRITISH JOURNAL OF PHARMACOLOGY,GB,BASINGSTOKE, HANTS, vol. 121, no. 8, 1 January 1997 (1997-01-01), pages 1513-1522, XP002043785 ISSN: 0007-1188
- WETZEL C H ET AL: "Use of gabapentin in pain management" ANNALS OF PHARMACOTHERAPY, vol. 31, no. 9, September 1997 (1997-09), pages 1082-1083, XP002101739 ISSN: 1060-0280 cited in the application

## Description

### BACKGROUND OF THE INVENTION

### 1. Field Of The Invention

The present invention relates to the use of analogs of glutamic acid and gamma-aminobutyric acid (GABA) for treatment of pain associated with renal colic.

### 2. Description of Related Art

GABA analogs are known agents useful in antiseizure therapy for central nervous system disorders such as epilepsy, Huntington's chorea, cerebral ischemia, Parkinson's disease, tardive dyskinesia, and spasticity. It has also been suggested that the compounds can be used as antidepressants, anxiolytics, and antipsychotics. See WO 92/09560 (United States Serial Number 618,692 filed November 27, 1990) and WP 93/23383 (United States Serial Number 886,080 filed May 20, 1992).

WO 97/33858 teaches that compounds related to gabapentin are useful for treating epilespy, faintness attacks, hypokinesia, cranial disorders, neurodegenerative disorders, depression, anxiety, panic, pain, and neuropathological disorders. WO 97/33858 does not specify what forms of pain are treated.

Additionally, the compounds of the invention are known for treatment of neuropathic pain. For example, see Rosner H; Rubin L; Kestenbaum A., Gabapentin adjunctive therapy in neuropathic pain states. Clin J Pain, 1996 Mar, 12:1, 56-8; Segal AZ; Rordorf G., Gabapentin as a novel treatment for postherpetic neuralgia. Neurology, 1996 Apr, 46:4, 1175-6; Wetzel CH; Connelly JF., Use of gabapentin in pain management. Ann Pharmacother, 1997 Sep, 31:9, 1082-3; Zapp JJ., Postpoliomyelitis pain treated with gabapentin [letter]. Am Fam Physician, 1996 Jun, 53:8, 2442, 2445; Cheville A, et al., Neuropathic pain in radiation myelopathy:a case report. Program book, American Pain Society (14th Annual Scientific Meeting). Abstract #95823, p. A-115; Sist T; Filadora V; Miner M; Lema M., Gabapentin for idiopathic trigeminal neuralgia: report of two cases. Neurology, 1997 May, 48:5, 1467; Waldman SD, Tutorial 28: Evaluation and Treatment of Trigeminal Neuralgia. Pain Digest (1997) 7:21-24; Mellick LB; Mellick GA., Successful treatment of reflex sympathetic dystrophy with gabapentin [letter]. Am J Emerg Med, 1995 Jan, 13:1, 96; Mellick GA; Seng MI., The use of gabapentin in the treatment of reflex sympathetic dystrophy and a phobic disorder. Am J Pain Manage 1995; 5:7-9; Mellick GA; Mellicy LB; Mellick LB., Gabapentin in the management of reflex sympathetic dystrophy [letter]. J Pain Symptom Manage, 1995 May, 10:4, 265-6; Mellick GA; Mellick LB., Reflex sympathetic dystrophy treated with gabapentin. Arch Phys Med Rehabil, 1997 Jan, 78:1, 98-105 and Mackin GA., Medical and pharmacologic management of upper extremity neuropathic pain syndromes. J Hand Ther, 1997 Apr-Jun, 10:2, 96-109.

Renal colic is commonly known as kidney stones. The passage of these crystalline fragments is so painful that it is proverbially known as the male equivalent of "child birth or labor." Patients are in such agony, that they are often rushed to hospital emergency rooms for treatment. While not life-threatening, the pain is so incapacitating that patients are often started on narcotic pain relievers.

The typical renal colic sufferer makes an initial visit to the hospital and is given i.m. morphine or equivalent for about 48 hours after diagnosis. Then the patient is sent home on oral narcotic analgesics and ESWL (lithotripsy) is performed within 1 week. Typically all of the fragments pass at home within two weeks. The pain intensity is universally on the top of all pain scales.

According to Urinary Calculi: ESWL, Endourology, and Medical Therapy, James E. Lingeman, et.al., 1994:51-71, the incidence of kidney stones is 335,000 patients/year (ie, those who are admitted to the ER because of renal colic). According to the Merck Manual, approximately 1 in every 1000 adults is hospitalized annually in the USA because of urinary stones (which equals about 200,000 per year).

About 80% of urinary calculi, or kidney stones, are composed of Ca, mainly calcium oxalate; 5% are uric acid; 2% cystine; and the remainder, magnesium ammonium phosphate. All of these stones are crystalline in structure, often with sharp edges that resemble small pieces of broken glass. Whether the stones pass through the ureter intact and spontaneously or in fragments following extracorporeal shock wave lithotripsy, extreme pain typically accompanies ureteral passage. This pain is often only partially relieved, even with the use of narcotic analgesics like morphine.

### SUMMARY OF THE INVENTION

This invention makes possible a method for treating renal colic comprising administering to a subject suffering from such pain an effective amount of a GABA analog. A preferred embodiment utilizes a cyclic amino acid compound of Formula I 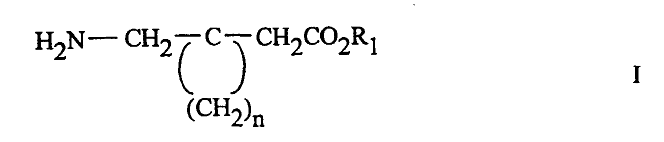 wherein R₁ is hydrogen or lower alkyl and n is an integer of from 4 to 6, and the pharmaceutically acceptable salts thereof. An especially preferred embodiment utilizes a compound of Formula I where R₁ is hydrogen and n is 4, which compound is 1-(aminomethyl)-cyclohexane acetic acid, known generically as gabapentin.

In another embodiment, the invention includes the use of a compound of Formula II for treating pain associated to renal colic. 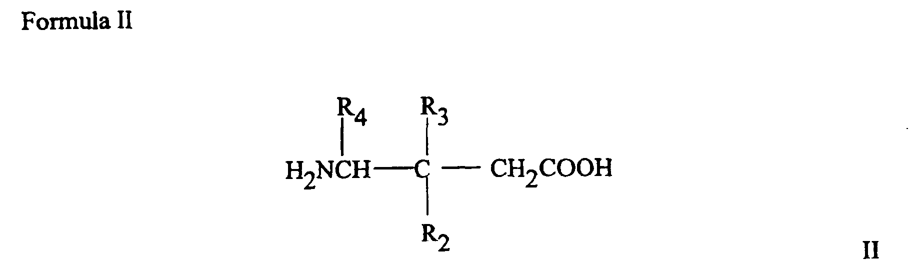 wherein R₂ is a straight or branched alkyl of from 1 to 6 carbon atoms, phenyl, or cycloalkyl having from 3 to 6 carbon atoms; R₃ is hydrogen or methyl; and R₄ is hydrogen, methyl, or carboxyl; or an individual enantiomeric isomer thereof; or a pharmaceutically acceptable salt thereof, in unit dosage form, to a mammal in need of said treatment.

Preferred compounds of the invention are those wherein R₄ and R₃ are hydrogen, and R₂ is -(CH₂)₀₋₂-i C₄H₉ as an (R), (S), or (R,S) isomer.

The more preferred compounds of Formula II invention are (S)-3-(aminomethyl)-5-methylhexanoic acid and 3-aminomethyl-5-methyl-hexanoic acid, now known generically as pregabalin.

### DETAILED DESCRIPTION OF THE PREFERRED EMBODIMENTS

The use of this invention utilizes any GABA analog. A GABA analog is any compound derived from or based upon gamma-aminobutyric acid. The compounds are readily available, either commercially, or by synthetic methodology well-known to those skilled in the art of organic chemistry. The preferred GABA analogs to be utilized are cyclic amino acids of Formula I. These are described in U.S. Patent 4,024,175. Another preferred use comprises the GABA analogs of Formula II, and these are described in U.S. Patent 5,563,175.

All that is required to practice the medical use of this invention is to administer a GABA analog in an amount that is effective to treat pain associated with renal colic. Such anti-pain amount will generally be from about 1 to about 300 mg per kg of subject body weight. Typical doses will be from about 10 to about 5000 mg per day for an adult subject of normal weight. It is expected that common doses that might be administered for renal colic could be from 100 mg three times a day up to 600 mg four times a day. Commercially available capsules of 100 mg, 300 mg and 400 mg of gabapentin can be administered. Alternate forms include liquids and film-coated tablets.

If a compound of Formula II , such as pregabalin is used, the dosage level is one sixth that of gabapentin. The dosage range for pregabalin is from about 0.15 mg to about 50 mg per kg per day of subject body weight. Typical dosages for pregabalin will be from about 1.6 mg to about 840 mg per day with individual dosages ranging from abut 0.15 mg to about 65 mg per dose.

The compounds of the present invention may form pharmaceutically acceptable salts with both organic and inorganic acids or bases. For example, the acid addition salts of the basic compounds are prepared either by dissolving the free base in aqueous or aqueous alcohol solution or other suitable solvents containing the appropriate acid and isolating the salt by evaporating the solution. Examples of pharmaceutically acceptable salts are hydrochlorides, hydrobromides, hydrosulfates, etc. as well as sodium, potassium, and magnesium, etc. salts.

The compounds of the Formula II can contain one or several asymmetric carbon atoms. The invention includes the individual diastereomers or enantiomers, and the mixtures thereof. The individual diastereomers or enantiomers may be prepared or isolated by methods already well-known in the art.

Pharmaceutical compositions of the compound of the present invention or its salts are produced by formulating the active compound in dosage unit form with a pharmaceutical carrier. Some examples of dosage unit forms are tablets, capsules, pills, powders, aqueous and nonaqueous oral solutions and suspensions, and parenteral solutions packaged in containers containing either one or some larger number of dosage units and capable of being subdivided into individual doses.

Some examples of suitable pharmaceutical carriers, including pharmaceutical diluents, are gelatin capsules; sugars such as lactose and sucrose; starches such as com starch and potato starch, cellulose derivatives such as sodium carboxymethyl cellulose, ethyl cellulose, methyl cellulose, and cellulose acetate phthalate; gelatin; talc; stearic acid; magnesium stearate; vegetable oils such as peanut oil, cottonseed oil, sesame oil, olive oil, corn oil, and oil of theobroma; propylene glycol, glycerin; sorbitol; polyethylene glycol; water; agar; alginic acid; isotonic saline, and phosphate buffer solutions; as well as other compatible substances normally used in pharmaceutical formulations. The compositions of the invention can also contain other components such as coloring agents, flavoring agents, and/or preservatives. These materials, if present, are usually used in relatively small amounts. The compositions can, if desired, also contain other therapeutic agents.

The percentage of the active ingredients in the foregoing compositions can be varied within wide limits, but for practical purposes it is preferably present in a concentration of at least 10% in a solid composition and at least 2% in a primary liquid composition. The most satisfactory compositions are those in which a much higher proportion of the active ingredient is present.

Routes of administration of the subject compound or its salts are oral or parenteral. For example, a useful intravenous dose is between 5 and 50 mg and a useful oral dosage is between 20 and 800 mg. The dosage is within the dosing range used in treatment of pain or as would be with the needs of the patient as described by the physician.

A unit dosage form of the GABA analog to be used in this invention may also comprise other compounds useful in the treatment of pain. These could include the urinary analgesic, phenazopyridine, as well as systemic analgesics.

The advantages of using the compounds of Formula I and II, especially gabapentin and pregabalin, in the instant invention include the relatively nontoxic nature of the compounds, the ease of preparation, the fact that the compounds are well-tolerated, and the ease of IV administration of the drugs. Gabapentin has few interactions with major classes of drugs since it is not metabolized in the liver, but rather excreted unchanged from the body. Further, the drugs are not metabolized in the body. The subjects treated with the method of the present invention are mammals, including humans.

While not wishing to be bound by any theory, the present invention appears to work because GABA analogs have been proposed to work both peripherally and centrally to relieve pain and to interact with a specific receptor in calcium channels. This calcium channel interaction is of particular interest when considering its potential role in renal colic, since calcium channel antagonists such as nifedipine have been shown to relieve the pain and promote passage of renal calculi through a vasodilatory effect on the ureter smooth muscle. This action of GABA analogs, specifically gabapentin, is confirmed, in part, by a documented low incidence of peripheral edema and vasodilatation. Thus, gabapentin could have a dual mechanism in the relief of acute renal colic through its interference with central and peripheral pain pathways in addition to its potential to provide ureter smooth muscle relaxation. Based on this possible dual mechanism gabapentin provides superior pain relief for renal colic relative to existing analgesics.

## Claims

1. Use of a GABA analog for the preparation of a pharmaceutical composition for treating a mammal suffering from pain associated with renal colic.

2. The use according to claim 1, wherein the GABA analog is the compound according to Formula I: 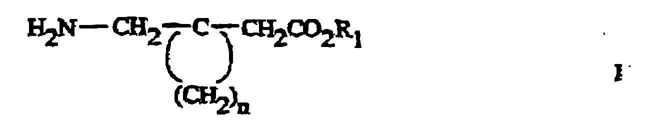 wherein R₁ is hydrogen or lower alkyl and n is an integer of from 4 to 6, and the pharmaceutically acceptable salts thereof.

3. The use according to claim 2, wherein Formula I comprises gabapentin.

4. The use according to claim 2, comprising from about 10 mg to about 400 mg of Formula I.

5. The use according to claim 3, comprising from about 10 mg to about 400 mg of gabapentin.

6. The use according to claim 1, wherein the GABA analog is a compound according to Formula II: 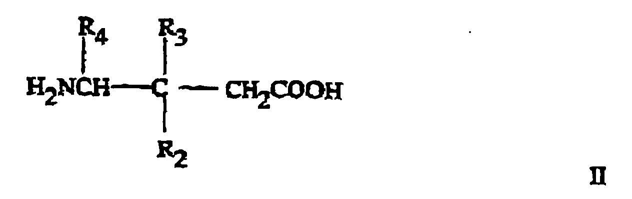 wherein R₂ is a straight or branched alkyl of from 1 to 6 carbon atoms, phenyl, or cycloalkyl having from 3 to 6 carbon atoms; R₃ is hydrogen or methyl; and R₄ is hydrogen, methyl, or carboxyl.

7. The use according to claim 6, wherein Formula II comprises pregabalin.

8. The use according to claim 6, comprising from about 0, 15 mg to about 65 mg of Formula II.

9. The use according to claim 7, comprising from about 0,15 mg to about 65 mg of pregabalin.

## Patentansprüche

1. verwendung eines GABA-Analogons zur Herstellung einer pharmazeutischen Zusammensetzung zur Behandlung eines Säugers, der an mit einer Nierenkolik in Verbindung stehendem Schmerz leidet.

2. Verwendung nach Anspruch 1, wobei das GABA-Analogon die Verbindung gemäß der Formel I: 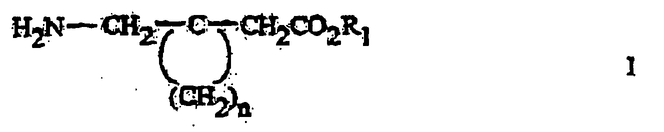 worin R₁ Wasserstoff oder Niederalkyl bedeutet und n eine ganze Zahl von 4 bis 6 ist, und die pharmazeutisch akzeptablen Salze derselben bedeutet.

3. verwendung nach Anspruch 2, wobei die Formel I Gabapentin umfasst.

4. Verwendung nach Anspruch 2, die etwa 10 mg bis etwa 400 mg der Formel I umfasst.

5. Verwendung nach Anspruch 3, die etwa 10 mg bis etwa 400 mg Gabapentin umfasst.

6. Verwendung nach Anspruch 1, wobei das GABA-Analogon eine Verbindung gemäß der Formel II: 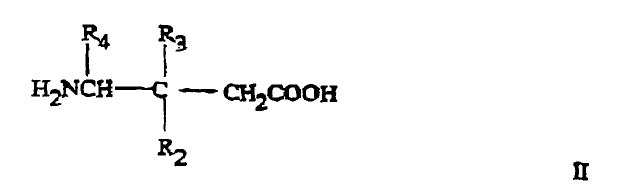 worin R₂ ein gerades oder verzweigtes Alkyl mit 1 bis 6 Kohlenstoffatomen, Phenyl oder Cycloalkyl mit 3 bis 6 Kohlenstoffatomen bedeutet; R₃ Wasserstoff oder Methyl bedeutet; und R₄ Wasserstoff, Methyl oder Carboxyl bedeutet, ist.

7. Verwendung nach Anspruch 6, wobei die Formel II Pregabalin umfasst.

8. Verwendung nach Anspruch 6, die etwa 0,15 mg bis etwa 65 mg der Formel II umfasst.

9. Verwendung nach Anspruch 7, die etwa 0,15 mg bis etwa 65 mg Pregabalin umfasst.

## Revendications

1. Utilisation d'un analogue du GABA pour la préparation d'une composition pharmaceutique permettant le traitement d'un mammifère souffrant de douleurs associées à une colique néphrétique.

2. Utilisation selon la revendication 1, dans laquelle l'analogue de GABA est le composé selon la Formule I: 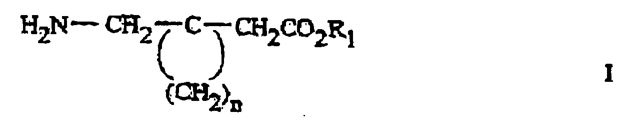 dans laquelle R₁ représente un atome d'hydrogène ou un groupe alkyle inférieur et n représente un nombre entier valant de 4 à 6, et ses sels pharmaceutiquement acceptables.

3. Utilisation selon la revendication 2, dans laquelle la Formule I comprend de la gabapentine.

4. Utilisation selon la revendication 2, comprenant de 10 mg environ à 400 mg environ de la Formule I.

5. Utilisation selon la revendication 3, comprenant de 10 mg environ à 400 mg environ de gabapentine.

6. Utilisation selon la revendication 1, dans laquelle l'analogue de GABA est un composé selon la Formule II: 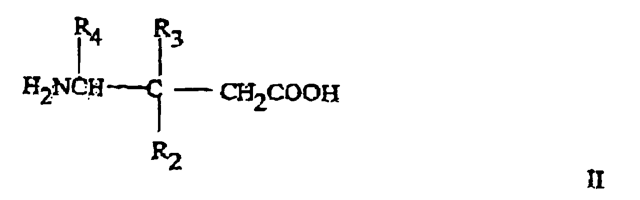 dans laquelle R₂ représente un groupe alkyle linéaire ou ramifié de 1 à 6 atomes de carbone, un groupe phényle, ou un groupe cycloalkyle comportant de 3 à 6 atomes de carbone ; R₃ représente un atome d'hydrogène ou un groupe méthyle ; et R₄ représente un atome d'hydrogène, un groupe méthyle, ou un groupe carboxyle.

7. Utilisation selon la revendication 6, dans laquelle la Formule II comprend de la prégabaline.

8. Utilisation selon la revendication 6, comprenant de 0,15 mg environ à 65 mg environ de la Formule II.

9. Utilisation selon la revendication 7, comprenant de 0,15 mg environ à 65 mg environ de prégabaline.
